# EUROPEAN PATENT SPECIFICATION

(11) **EP 2 764 888 B1**
(45) Date of publication and mention of the grant of the patent: **13.07.2016**
(21) Application number: 13154151.8
(22) Date of filing: 06.02.2013
(51) Int. Cl.: A61N 1/05

(54) **A perineal probe**
Dammsonde
Sonde périnéale

(43) Date of publication of application: 13.08.2014
(73) Proprietor: Beacmed S.r.L., 27040 Portalbera (Pavia) (IT)
(72) Inventor: Bozzarelli, Pier Luigi, I - 27040 Portalbera (Pavia) (IT)
(74) Representative: Lunati & Mazzoni S.r.L.

(56) References cited:
- EP-A1- 2 322 110
- WO-A1-2007/136266
- WO-A2-2011/159906
- US-A1- 2005 228 316

## Description

The present description relates to a perineal probe, in particular for the treatment of incontinence. The invention is set out in the appended claims.

Various perineal probes used for different purposes, and in particular for the treatment and re-education of incontinence, are currently known. Said incontinence, whether faecal or urinary, may be caused by many different reasons and factors and be due to the malfunctioning of a plurality of local groups of muscles.

Some probes are described in patent applications: US-A-2005/0228316. EP-A-2 322 110. WO-A-2007/136266 and WO-A-2011/159906. In particular, US-A-2005/0228316 discloses a perineal probe according to the preamble of claim 1.

Current methods of re-education for incontinence involve the use of vaginal or anal probes to detect electromyographic activity using EMG (electromyography) or to administer electrical stimulation, or to measure the pressure in the vaginal or anal cavity.

These probes are generally characterised by metal or metallised electrodes, with a circularly symmetric structure. Said probes also come in different types: flat, with unilateral electrodes, or even with a hole in the middle.

However, the perineal probes of the type known in the prior art have some important drawbacks.

In particular, the probes known in the prior art do not permit the precise selection of the groups of muscles to be treated.

As a consequence, said probes may not stimulate the muscles properly, delaying or undoing the benefits of the treatment.

In this situation the technical purpose of the present description is to devise a perineal probe able to overcome the drawbacks mentioned above.

Within the sphere of said technical purpose, an important aim of the disclosure is to provide a perineal probe that permits precise and localised treatments of the groups of muscles to be treated.

A further purpose of the description is to obtain a simple and economical perineal probe.

The technical purpose and specific aims are achieved with a perineal probe as claimed in the appended Claim 1. Preferred embodiments are described in the dependent claims. The embodiments, aspects or examples of the present description that do not fall within the scope of said claims are provided for illustrative purposes only do not form part of the present invention.

The characteristics and advantages of the invention are clearly evident from the following detailed description of a preferred embodiment thereof, with reference to the accompanying drawings, in which:

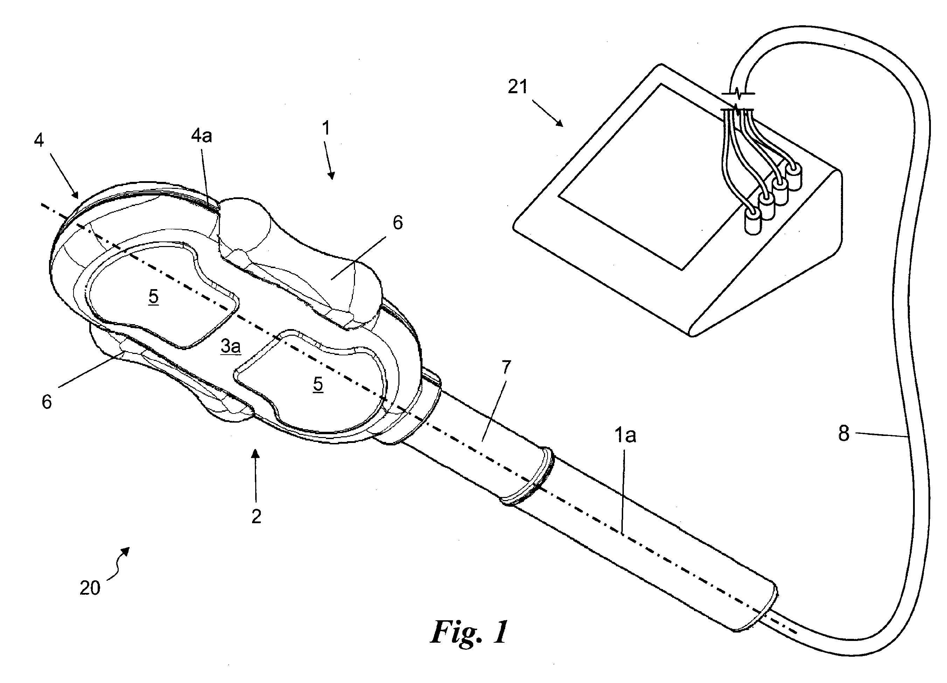

**Fig. 1** schematically illustrates the perineal probe connected to control means;
**Fig. 2** is a scale drawing showing an axonometric view partially from below of the perineal probe according to the description;
**Fig. 3** is a scale drawing showing an axonometric view partially from above of the perineal probe according to the description;
**Fig. 4** is a scale drawing of the perineal probe in a view from the bottom;
**Fig. 5** is a scale drawing of the perineal probe in a view from above;
**Fig. 6** is a scale drawing of the perineal probe in a view from the side; and
**Fig. 7** is a scale drawing of the perineal probe in a view from the front.

With reference to said drawings, reference numeral 1 globally denotes the perineal probe according to the description. It is suitable to be connected to control means **21** of said perineal probe 1 suitable to control said probe and/or to receive information from said probe and, if necessary, analyse or simply transmit said information. The perineal probe 1 and the control means 21 constitute an apparatus **20.**

The perineal probe 1 comprises a main axis **1a** which coincides with the direction in which the probe 1 is inserted into the human body and also with the main direction of extension of the perineal probe 1.

In brief, the probe comprises a main body **2,** comprising electrodes, which are described below, and a supporting rod **7,** suitable to move and support the main body 2 and extending along said main axis 1 a.

The main body 2 comprises a continuous lower surface **3a,** an upper surface **3b,** opposite the lower surface 3a and a lateral surface **4,** extending between the lower surface 3a and the upper surface 3b and comprising two opposite main portions **4a** substantially parallel to the main axis 1 a.

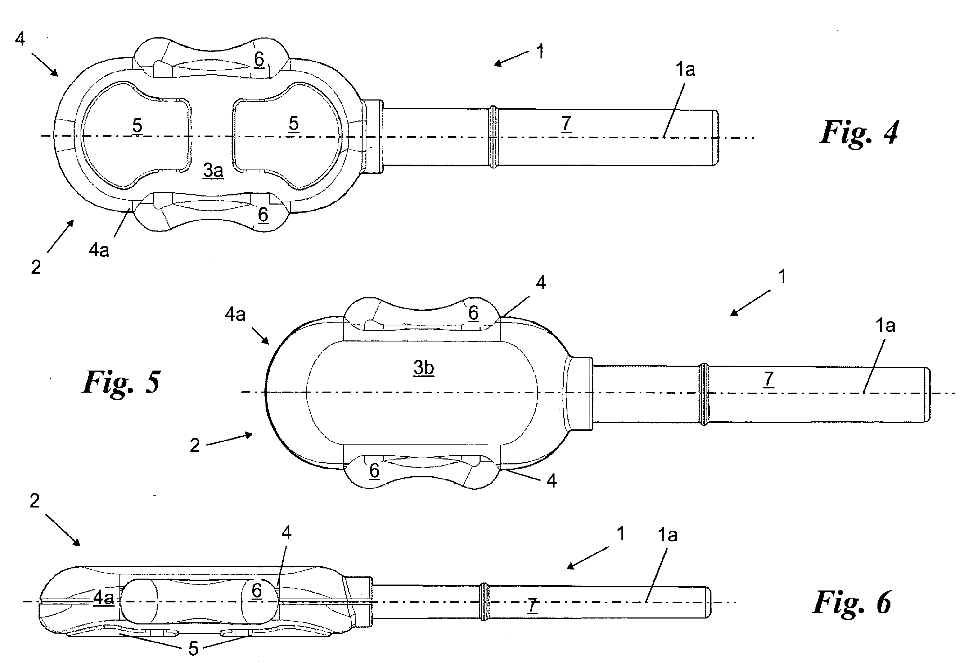

More in detail, the lower 3a and upper 3b surfaces may be substantially identical. The lower surface 3a is preferably substantially flat and substantially parallel to the main axis 1 a. Alternatively it may be convex or otherwise shaped. It is also substantially continuous, i.e. with no holes or other forms of discontinuity. Clearly, the term substantially refers to the fact that there may be some very slight breaks given by the space between the seats of the electrodes and the electrodes themselves, or between different portions of the body 2, etc. Furthermore, the lower 3a and upper 3b surfaces have a shape that is elongated, along the plane, and preferably ellipsoidal with two partially rectilinear sides, as illustrated in Figs. 4 and 5.

The lateral surface 4 joins the two lower 3a and upper 3b surfaces with curved walls, preferably with a substantially constant curvature radius. The length of said main body 2, in the direction of the main axis 1a, is preferably comprised between 3 cm and 7 cm, its width, in the direction perpendicular to the main axis 1 a and parallel to the surface 3a, is preferably comprised between 1 cm and 3 cm, while the height, perpendicular to the width and the length, is comprised between 0.3 cm and 2 cm.

The main body 2 also comprises at least two lower electrodes **5,** arranged on the lower surface 3a and reciprocally separated along the main axis 1a, and at least two lateral electrodes **6** arranged along the main portions 4a of the lateral surface 4.

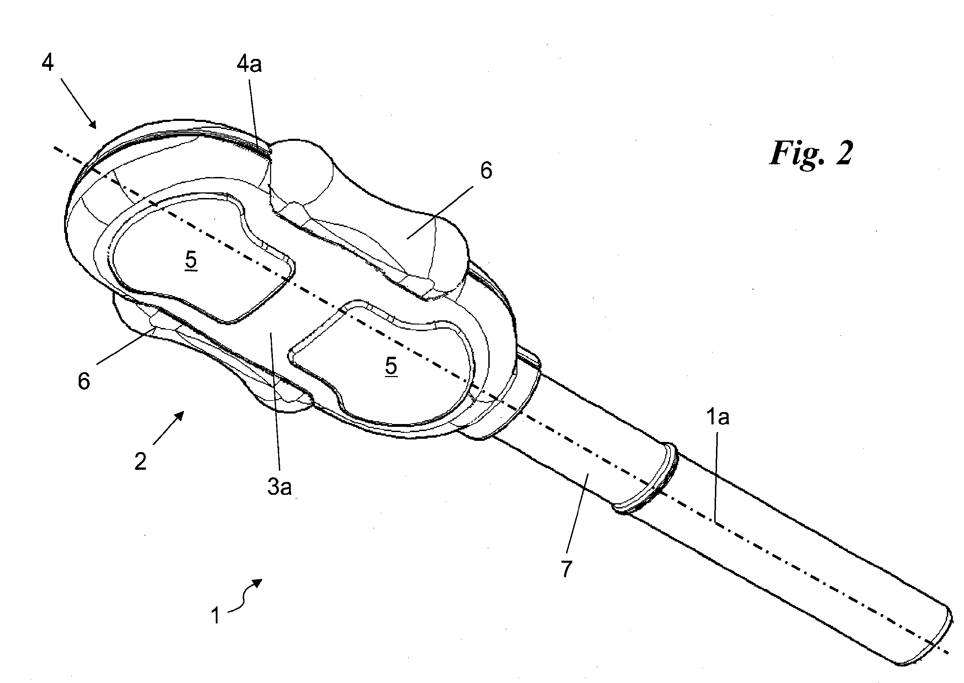

In particular the lower electrodes 5 have an average diameter comprised between 5 mm and 2 cm and a partially circular and partially rectangular shape, as illustrated in Figs. 2 and 4. Said lower electrodes 5 are also reciprocally separated by a distance comprised between 0.8 and 0.3 times the size of said average diameter of the lower electrodes 5. Furthermore they are preferably flat and protrude by a length comprised between 0.5 mm and 2 mm from the lower surface 3a. Lastly, the lower electrodes 5 are preferably aligned in parallel with the axis 1 a and centred on said axis.

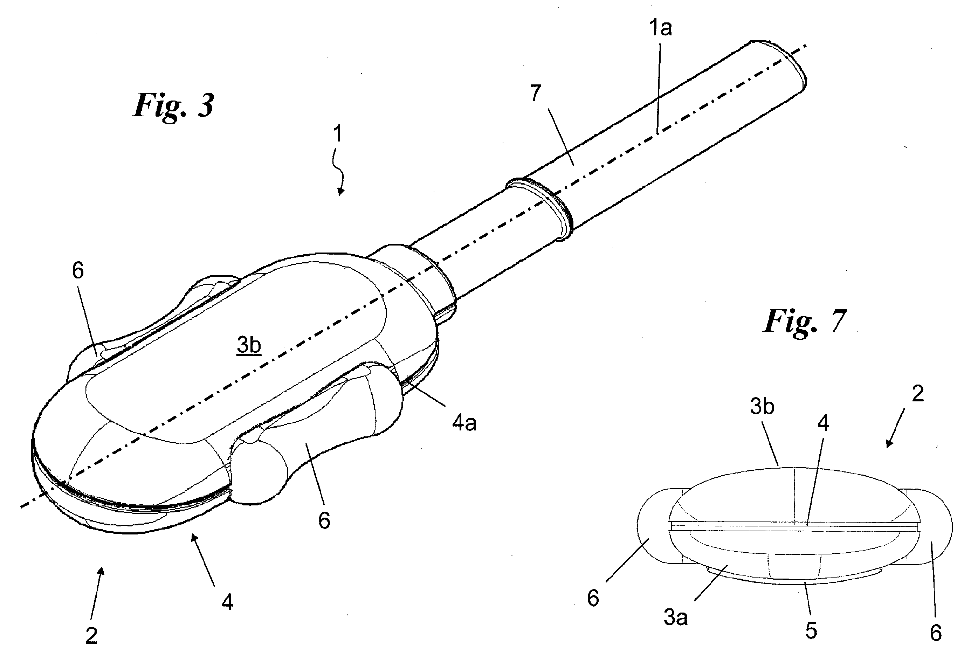

The lateral electrodes 6 extend primarily in a direction parallel to the main axis 1 a and are saddle shaped, i.e. having a minimum distance from the main axis 1 in a central portion thereof, as illustrated in Figs. 2 and 3. They are also of a length comprised between 1 cm and 3 cm and are specularly symmetrical and reciprocally aligned along the main axis 1 a. More in detail, the narrowest part of the saddle shape is arranged in correspondence with the space between the lower electrodes 5, as illustrated in Fig. 2.

Moreover, the perineal probe 1 may comprise at least one upper electrode arranged on the upper surface 3b and not illustrated in the accompanying drawings.

The supporting rod 7 mechanically connected to the main body 2 comprises electrical connections 8 connected to the electrodes 5 and 6 and connectable to the control means 21 of the perineal probe 1.

In particular, the connections 8 comprise electrical cables inside the rod 7 and a board with printed circuits or similar components inside the main body 2. Moreover each single electrode 5 and 6 is provided with an independent electrical connection 8 electrically isolated from the other electrical connections 8. Said solution permits the independent control or receipt of information for each pair of electrodes. The electrical connections 8 also comprise flexible cables that extend beyond the rod 7.

Lastly, the apparatus 20, as described previously, also comprises control means 21 suitable to be connected to the electrical connections 8. They are suitable to control the lower electrodes 5 and the lateral electrodes 6 simultaneously and in a reciprocally independent manner.

Structurally, the main body 2 consists of two shells, preferably partially symmetrical polymeric shells, reciprocally glued or welded. Each shell includes a surface 3a or 3b. Moreover, the lower shell comprises the seats for the lower electrodes 6, consisting of recesses provided with a passage for the electrodes towards the centre of the body 2, where the electrical connections 8 are present. The lower shell also comprises a portion of seats for the lateral electrodes. The upper shell instead comprises the latter.

In the centre of the body 2 there is a printed circuit board PCB which includes the electrical connections 8. Said board also connects the electrodes 5 and 6 preferably by means of joints and/or welding.

The rod 7 is made of a polymeric material over-moulded on the electrical connections 8 consisting of electrical cables.

The perineal probe 1 according to the invention works in the following way. The physician or his/her assistant inserts the probe into the patient's vaginal or anal cavity and controls it using the control means 21.

In particular, the probe 1 is arranged so that the two branches (rh/lh) of the pubococcygeus muscle rest on the narrowest parts of the lateral saddle-shaped electrodes 6.

In particular, as is known, when any muscle contracts naturally it produces electricity (EMG) which can also be measured using two electrodes arranged at a certain distance along the muscle; vice versa, a muscle contracts artificially when stimulated by an electrical current applied thereto through electrodes arranged along said muscle.

Re-education of incontinence is therefore possible by means of said impulses supplied by the control means 21, transmitted by the electrical connections 8 and by the electrodes 5 and 6. On the contrary, it is also possible to receive signals that monitor any muscular contractions.

In particular, the lower electrodes 5 selectively stimulate the puborectalis muscle, while the lateral electrodes 6 selectively stimulate the pubococcygeus.

Said technical effect is primarily due to the position of the electrodes 5 and 6 and also, to a lesser extent, to their shape and size, their position along the main body 2 and the position of the probe 1 inside the human body, obtained thanks to the saddle shape of the lateral electrodes 6.

The perineal probe according to the present invention achieves some important advantages.

Thanks to said technical effects it is possible to deliver precise therapies or treatments suited to the patient's problem or physical and muscular structure.

The selective stimulation of different muscles allows the physician to choose different stimulation programs for the electrodes, in particular different in terms of frequency, intensity, waveform, etc. The different electrode stimulations can also be simultaneous. The invention is set out in the following claims*.*

## Claims

1. Perineal probe (1) comprising a main axis (1a) coinciding with the direction of insertion of said probe (1) and a main body (2), said main body (2) comprising: a continuous lower surface (3a), an upper surface (3b), opposite said lower surface (3a), a lateral surface (4), extending between said lower surface (3a) and said upper surface (3b) and comprising two opposite main portions (4a) substantially parallel to said main axis (1 a), said perineal probe (1) also comprising two lateral saddle-shaped electrodes (6) arranged along said main portions (4a) of said lateral surface (4) **characterised in that** said lower surface (3a) is substantially flat and **in that** it comprises two lower electrodes (5), having an average diameter comprised between 5 mm and 2 cm, arranged on said lower surface (3a), reciprocally separated along said main axis (1a) and aligned in parallel with said main axis (1a) and centred on said main axis (1a).

2. Perineal probe (1) as claimed the preceding claim, wherein said lower electrodes (5) are reciprocally separated by a distance comprised between 0.8 and 0.3 times the size of said average diameter of said lower electrodes (5).

3. Perineal probe (1) as claimed in one or more of the preceding claims, wherein the length of said lateral electrodes (6) is comprised between 1 cm and 3 cm.

4. Perineal probe (1) as claimed in claim 5, wherein said lateral electrodes (6) are specularly symmetrical and reciprocally aligned along said main axis (1a) and wherein said saddle shape comprises a narrowest part in correspondence with said space separating said lower electrodes (5).

5. Perineal probe (1) as claimed in one or more of the preceding claims, comprising at least one upper electrode arranged on said upper surface (3b).

6. Perineal probe (1) as claimed in one or more of the preceding claims, comprising a supporting rod (7) including electrical connections (8) to said electrodes (5, 6) and wherein said electrical connections are connectable to control means (21) of said perineal probe (1).

7. Perineal probe (1) as claimed in claim 8, wherein each of said electrical connections (8) is specific for a single electrode (5, 6) and is electrically separated from the other electrical connections (8).

8. Perineal probe (1) as claimed in one or more of the preceding claims, wherein said lower surface (3a) is substantially parallel to said main axis (1a).

9. Apparatus (20) including a perineal probe (1) as claimed in one or more of the preceding claims and control means of said perineal probe (1), wherein said control means (21) are suitable to control said lower electrodes (5) and said lateral electrodes (6) simultaneously and in a reciprocally independent manner.

## Patentansprüche

1. Dammsonde (1), die eine mit der Einführrichtung der genannten Sonde (1) übereinstimmende Hauptachse (1 a) und einen Hauptkörper (2) umfasst, wobei der genannte Hauptkörper (2) Folgendes umfasst: eine durchgehende untere Fläche (3a), eine obere Fläche (3b), die der genannten unteren Fläche (3a) gegenüberliegt, eine Seitenfläche (4), die zwischen der genannten unteren Fläche (3a) und der genannten oberen Fläche (3b) verläuft und zwei Hauptabschnitte (4a) umfasst, die der genannten Hauptachse (1a) gegenüberliegen und im Wesentlichen zu ihr parallel sind, wobei die genannte Dammsonde (1) außerdem zwei seitliche Elektroden (6) mit einer Sattelform umfasst, die entlang der genannten Hauptabschnitte (4a) der genannten Seitenfläche (4) angeordnet sind, **dadurch gekennzeichnet, dass** die genannte untere Fläche (3a) im Wesentlichen flach ist und dadurch, dass sie zwei untere Elektroden (5) mit einem durchschnittlichen Durchmesser zwischen 5 mm und 2 cm umfasst, die auf der genannten unteren Fläche (3a) angeordnet und voneinander entlang der genannten Hauptachse (1 a) getrennt und parallel zu der genannten Hauptachse (1 a) ausgerichtet und auf der genannten Hauptachse (1 a) zentriert sind.

2. Dammsonde (1) nach einem oder mehreren der vorangegangenen Ansprüche, bei der die genannten unteren Elektroden (5) voneinander in einem Abstand zwischen 0,8 und 0,3 Mal der Größe des genannten durchschnittlichen Durchmessers der genannten unteren Elektroden (5) getrennt sind.

3. Dammsonde (1) nach einem oder mehreren der vorangegangenen Ansprüche, bei der die genannten seitlichen Elektroden (6) eine Länge zwischen 1 cm und 3 cm aufweisen.

4. Dammsonde (1) nach Anspruch 5, bei der die genannten seitlichen Elektroden (6) spiegelsymmetrisch und jeweils entlang der genannten Hauptachse (1 a) ausgerichtet sind und bei der die genannte Sattelform einen Mindestabstand der genannten Trennung zwischen den genannten unteren Elektroden (5) aufweist.

5. Dammsonde (1) nach einem oder mehreren der vorangegangenen Ansprüche, die mindestens eine auf der genannten oberen Fläche (3b) angeordnete obere Elektrode umfasst.

6. Dammsonde (1) nach einem oder mehreren der vorangegangenen Ansprüche, die eine Haltestange (7) mit elektrischen Anschlüssen (8) an die genannten Elektroden (5, 6) umfasst und bei der die genannten elektrischen Anschlüsse an Steuervorrichtungen (21) der genannten Dammsonde (1) angeschlossen werden können.

7. Dammsonde (1) nach Anspruch 8, bei der jeder der genannten elektrischen Anschlüsse (8) für eine einzelne Elektrode (5, 6) bestimmt und elektrisch von den anderen genannten elektrischen Anschlüssen (8) getrennt ist.

8. Dammsonde (1) nach einem oder mehreren der vorangegangenen Ansprüche, bei der die genannte untere Fläche (3a) im Wesentlichen parallel zu der genannten Hauptachse (1 a) verläuft.

9. Apparat (20), der eine Dammsonde (1) nach einem der vorangegangenen Ansprüche und Steuervorrichtungen der genannten Dammsonde (1) umfasst, bei dem die genannten Steuervorrichtungen (21) geeignet sind, gleichzeitig und voneinander unabhängig die genannten unteren Elektroden (5) und die genannten seitlichen Elektroden (6) zu steuern.

## Revendications

1. Sonde périnéale (1) comprenant un axe principal (1 a) coïncidant avec la direction d'insertion de ladite sonde (1) et un corps principal (2), ledit corps principal (2) comprenant : une surface inférieure (3a) continue, une surface supérieure (3b), opposée à ladite surface inférieure (3a), une surface latérale (4), se développant entre ladite surface inférieure (3a) et ladite surface supérieure (3b) et comprenant deux parties principales (4a) opposées et essentiellement parallèles audit axe principal (1 a), ladite sonde périnéale (1) comprenant en outre deux électrodes latérales (6) ayant la forme d'une selle placée le long desdites parties principales (4a) de ladite surface latérale (4) **caractérisée en ce que** ladite surface inférieure (3a) est essentiellement plate et **en ce qu'**elle comprend deux électrodes inférieures (5) ayant un diamètre moyen compris entre 5 mm et 2 cm, disposées sur ladite surface inférieure (3a) et réciproquement séparées le long dudit axe principal (1 a) et alignées en parallèle sur ledit axe principal (1 a) et centrées sur ledit axe principal (1 a).

2. Sonde périnéale (1) selon une ou plusieurs des revendications précédentes, dans laquelle lesdites électrodes inférieures (5) sont séparées réciproquement d'une distance comprise entre 0,8 et 0,3 fois la grandeur dudit diamètre moyen desdites électrodes inférieures (5).

3. Sonde périnéale (1) selon une ou plusieurs des revendications précédentes, dans laquelle lesdites électrodes latérales (6) ont une longueur comprise entre 1 cm et 3 cm.

4. Sonde périnéale (1) selon la revendication 5, dans laquelle lesdites électrodes latérales (6) sont symétriques de façon spéculaire et réciproquement alignées le long dudit axe principal (1 a) et dans laquelle ladite forme à selle comprend un minimum de correspondance de ladite séparation entre lesdites électrodes inférieures (5).

5. Sonde périnéale (1) selon une ou plusieurs des revendications précédentes, comprenant au moins une électrode supérieure disposée sur ladite surface supérieure (3b).

6. Sonde périnéale (1) selon une ou plusieurs des revendications précédentes comprenant une tige de support (7) incluant des connexions électriques (8) auxdites électrodes (5, 6) et dans laquelle lesdites connexions électriques peuvent être connectées à des moyens de commande (21) de ladite sonde périnéale (1).

7. Sonde périnéale (1) selon la revendication 8, dans laquelle chacune desdites connexions électriques (8) est destinée à une seule électrode (5, 6) et est électriquement séparée des autres dites connexions électriques (8).

8. Sonde périnéale (1) selon une ou plusieurs des revendications précédentes, dans laquelle ladite surface inférieure (3a) est essentiellement parallèle audit axe principal (1a).

9. Appareil (20) incluant une sonde périnéale (1) selon une ou plusieurs des revendications précédentes et des moyens de commande de ladite sonde périnéale (1), dans lequel lesdits moyens de commande (21) sont aptes à commander simultanément et réciproquement indépendamment lesdites électrodes inférieures (5) et lesdites électrodes latérales (6).
